# FASCICULE DE BREVET EUROPEEN

(11) **EP 0 682 918 B1**
(45) Date de publication et mention de la délivrance du brevet: **03.05.2000**
(21) Numéro de dépôt: 95420130.7
(22) Date de dépôt: 19.05.1995
(51) Int. Cl.: A61B 17/60

(54) **Dispositif d'ostéosynthèse pour rachis**
Vorrichtung zur Wirbelsäulenosteosynthese
Spinal osteosynthesis device

(30) Priorité: 20.05.1994 FR 9406428
(43) Date de publication de la demande: 22.11.1995
(73) Titulaire: Groupe Lepine, 69003 Lyon (FR); OBJECTIF IMPLANTS, 10000 Troyes (FR)
(72) Inventeur: Pfaifer, Patrick, F-69003 Lyon (FR); Dehais, Valéry, F-10000 Troyes (FR)
(74) Mandataire: Maureau, Philippe

(56) Documents cités:
- EP-A- 0 330 881
- EP-A- 0 443 892
- EP-A- 0 487 895
- EP-A- 0 514 303
- EP-A- 0 528 706
- WO-A-91/16020
- FR-A- 2 642 643

## Description

La présente invention concerne un dispositif d'ostéosynthèse pour rachis.

La chirurgie du rachis nécessite le rétablissement des courbures physiologiques, la stabilisation des vertèbres les unes sur les autres et l'équilibre au-dessus des membres inférieurs par l'intermédiaire du sacrum et du bassin. A l'échelon disco-vertébral, les courbures et les dimensions physiologiques impliquent des actions de distraction, de compression, de lordose ou de cyphose selon le niveau vertébral concerné. Le dispositif d'ostéosynthèse doit donc tenir compte de tous ces impératifs, et comprend généralement des éléments d'ancrage osseux venant se fixer dans les vertèbres et des tiges d'étayage du rachis venant se fixer sur les extrémités des éléments d'ancrage dépassant des vertèbres.

Un dispositif comprenant des éléments d'ancrage osseux sous forme de vis pédiculaires est connu par la demande internationale N° WO 91/16020. La tête de chaque vis est engagée et retenue dans un manchon, qui comprend deux branches latérales parallèles, délimitant entre elles un canal de réception de la tige d'étayage. Ce manchon reçoit un élément intermédiaire d'appui, comprenant deux petites extensions latérales, d'environ 3,3 mm de longueur (0,13 inch), aptes à venir en appui contre la tige. Le canal est fermé, après introduction de la tige, par un écrou vissé sur la paroi extérieure filetée des branches parallèles, cet écrou venant en appui sur lesdites extensions latérales, qui font légèrement saillie au-delà des extrémités du manchon.

L'implantation de ce type de dispositif a pour inconvénient d'occasionner des blessures aux tissus environnants, du fait de la proéminence de la clef de serrage engagée sur l'écrou et des mouvements de pivotement de cette clef. Ces blessures ne sont souhaitables ni pour le patient, ni pour le praticien compte tenu des saignements qu'elles peuvent occasionner, ces saignements venant gêner l'implantation.

Ce problème survient particulièrement dans le dispositif décrit par la demande internationale précitée, du fait du diamètre important du manchon, et donc de l'écrou.

De plus, ces dispositifs ne garantissent pas la parfaite immobilisation dans le temps des tiges d'étayage par rapport aux vis d'ancrage, compte tenu des sollicitations importantes et répétées qu'exercent les tiges sur les vis, et compte tenu du fait que les tiges ne sont fréquemment pas parfaitement perpendiculaires aux vis d'ancrage.

Il est également connu, notamment par la demande de brevet européen N° 0 528 706, de prévoir des vis pédiculaires dont les têtes comprennent les branches parallèles précitées ; d'engager des bagues autour de ces branches, ces bagues comprenant des lames transversales incurvées, s'étendant vers l'intérieur, destinées à venir en appui contre les tiges ; de visser des bouchons filetés sur les parois intérieures taraudées des branches, ces bouchons venant porter axialement sur les lames transversales incurvées pour serrer la tige ; puis de placer des capuchons de recouvrement sur les extrémités supérieures des branches.

Ce deuxième type de dispositif réduit les blessures occasionnées aux tissus environnants. Toutefois, certains dispositifs ont pour inconvénient d'être difficiles et longs à poser compte tenu des mises en place successives des différents éléments qu'ils comprennent. En effet, l'engagement et le positionnement correct de ces éléments est difficile en fond de cavité, surtout si des saignements plus ou moins abondants se produisent, d'autant plus que ces pièces ont des dimensions réduites. Tel est en particulier le cas de l'engagement des filetages des bouchons sur les parois taraudées des branches.

Il en résulte que les durées d'intervention sont importantes, ce qui n'est pas souhaitable pour le patient.

De plus, le positionnement par rapport à la bague des surfaces d'appui sur la tige, les dimensions de ces surfaces, ainsi que la façon dont ces surfaces sont rattachées à la bague font que la parfaite immobilisation dans le temps des tiges par rapport aux vis n'est pas garantie dans toutes les circonstances, pour les mêmes raisons que celles indiquées plus haut.

Certains dispositifs existants ont par ailleurs pour inconvénient d'être relativement volumineux, et donc d'être peu confortables pour le patient.

L'invention vise à remédier à l'ensemble de ces inconvénients, en fournissant un dispositif permettant d'assurer une parfaite immobilisation des tiges par rapport aux éléments d'ancrage, c'est-à-dire une immobilisation susceptible de résister dans le temps aux contraintes qu'exercent les tiges d'étayage sur les éléments d'ancrage, et d'assurer cette immobilisation dans toutes les situations, notamment lorsque les tiges ne sont pas parfaitement perpendiculaires aux éléments d'ancrage.

L'invention vise également à fournir un dispositif pouvant facilement et rapidement être mis en place, confortable pour le patient, et n'occasionnant pas de blessures aux tissus environnants.

Le dispositif auquel elle se rapporte comprend, de manière connue en soi, des éléments d'ancrage osseux et au moins une tige d'étayage, chaque élément d'ancrage présentant deux branches latérales parallèles qui délimitent entre elles un canal de réception de la tige d'étayage, et recevant une bague engagée autour des branches ainsi qu'un bouchon, vissable entre les branches.

Selon l'invention, le bouchon porte sur la bague lors de son vissage, et la bague comprend deux encoches diamétralement opposées, délimitant deux surfaces concaves de contact avec la tige, et au moins une extension en saillie radiale vers l'extérieur, faisant corps avec la bague, qui s'étend en majeure partie à l'extérieur du périmètre de la bague, largement au-delà des extrémités latérales des branches, et qui prolonge vers l'extérieur l'une au moins desdites surfaces de contact.

Le serrage du bouchon permet à la bague de serrer la tige entre elle et le fond du canal recevant la tige.

La bague vient ainsi au contact de la tige non pas sensiblement dans l'axe du vissage du bouchon, mais par une ou deux surfaces de contact latérales, faisant corps avec la bague, de dimensions importantes et distantes l'une de l'autre.

Le large appui obtenu permet d'assurer une parfaite immobilisation de la tige par rapport aux éléments d'ancrage osseux, susceptible de résister dans le temps aux contraintes répétées, notamment en torsion, qu'exerce la tige sur les éléments d'ancrage lors des différents mouvements du rachis, sans aucun risque de mouvements de la tige par rapport aux éléments d'ancrage.

Ces surfaces d'appui permettent également, par leurs dimensions et leur distance relative, d'assurer le bon positionnement de la tige dans le canal, sans porte-à-faux, même lorsque la tige n'est pas parfaitement perpendiculaire à l'élément d'ancrage.

Le dispositif n'occasionne aucune blessure aux tissus environnants lors de son implantation. En outre, il est peu volumineux et se trouve près des corps vertébraux après implantation, et est donc confortable pour le patient.

De préférence, le bouchon fileté est assemblé à la bague dans la position qu'il est destiné à occuper par rapport à celle-ci après mise en place sur le rachis.

La bague et le bouchon forment ainsi un ensemble unitaire qui peut être mis en place en une seule opération. En effet, la bague peut être engagée sur les branches de l'élément d'ancrage jusqu'à ce que le filet du bouchon rencontre le filet des parois taraudées des branches. Le bouchon est alors placé en position adéquate par rapport aux branches, prêt à être vissé.

Grâce à cette disposition, le dispositif est facile et rapide à mettre en place, même dans le fond de la cavité et en cas de saignements abondants. Un gain de temps tout-à-fait appréciable, de l'ordre de quarante minutes, peut être obtenu sur une intervention.

Selon une forme de réalisation préférée de l'invention, la bague comprend une paroi transversale, faisant corps avec elle et s'étendant diamétralement par rapport à elle, en dessus de la ou desdites surfaces de contact avec la tige par rapport à l'élément d'ancrage, le bouchon étant monté sur cette paroi.

Cette paroi permet une parfaite transmission à la bague des efforts de serrage exercés par le bouchon.

Avantageusement, les passages prévus de part et d'autre de la paroi transversale pour l'engagement des branches de l'élément d'ancrage au travers de la bague sont précisément ajustées à la section transversale de ces branches. Cet ajustement permet d'immobiliser complètement la bague en rotation sur les branches lors de son engagement, et d'obtenir, par ce simple engagement, un parfait positionnement des surfaces d'appui par rapport à l'élément d'ancrage, ainsi qu'un parfait positionnement axial du filet du bouchon par rapport au filet des parois taraudées des branches.

Avantageusement, la ou lesdites extensions se raccordent à la bague par des parois arrondies à rayon de courbure relativement important, permettant de bonnes transmissions et répartitions sur la bague des contraintes exercées par la tige.

De préférence, la tige et les surfaces de contact de la bague avec elle présentent des aspérités de surface venant intimement coopérer les unes avec les autres lors du serrage de la bague. Il peut notamment s'agir de reliefs en pointe de diamant qui s'interpénètrent, ou d'un moletage.

Avantageusement, les canaux des éléments d'ancrage, les surfaces de contact précitées et la ou les extensions de la bague sont conformés de manière à ce que leur axe ne soit pas perpendiculaire à l'axe de vissage du bouchon, afin de pouvoir venir s'appliquer sans porte-à-faux sur une tige non perpendiculaire à l'élément d'ancrage, plusieurs éléments d'ancrage ou bagues comprenant des canaux, surfaces de contact et extensions d'inclinaisons différentes par rapport audit axe étant prévus et pouvant être utilisés alternativement selon les cas.

Pour sa bonne compréhension, l'invention est à nouveau décrite ci-dessous, en référence au dessin schématique annexé représentant, à titre d'exemples non limitatifs, deux formes de réalisation préférées d'un élément d'ancrage osseux et de l'une des tiges que comprend le dispositif qu'elle concerne.
La figure 1 est une vue en perspective, sous différents angles, de cet élément d'ancrage, de cette tige et de l'ensemble bague-bouchon fileté par lequel cet élément et cette tige sont assemblés l'un à l'autre, selon une première forme de réalisation ;
la figure 2 est une vue en coupe passant par l'axe de ces différents éléments, après assemblage ;
la figure 3 est une vue dudit ensemble bague-bouchon fileté selon une deuxième forme de réalisation, et
la figure 4 est une vue en coupe passant par l'axe de la tige, de cet ensemble et de l'élément d'ancrage osseux, après assemblage à l'élément d'ancrage osseux.

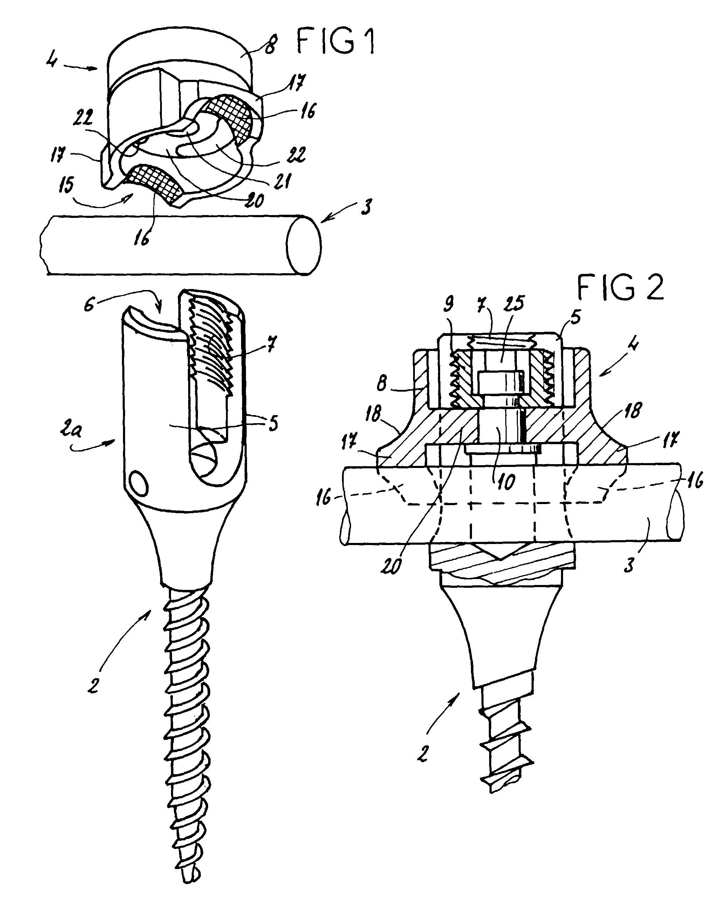

Les figures 1 et 2 représentent une vis pédiculaire 2, constituant l'élément d'ancrage osseux précité, une tige 3 d'étayage du rachis et un ensemble 4 permettant l'assemblage de la tige 3 à la vis 2.

La tête 2a de la vis 2 comprend deux branches latérales 5 délimitant entre elles un canal 6 de réception de la tige 3. Les branches 5 ont une section transversale en arc de cercle et comprennent un taraudage 7 aménagé dans leurs faces en regard.

Comme le montre plus particulièrement la figure 2, l'ensemble 4 comprend une bague 8, un bouchon fileté 9 et un rivet 10 d'assemblage de la bague 8 et du bouchon fileté 9.

La bague 8 comprend deux encoches 15 diamétralement opposées, délimitant deux surfaces concaves 16 de contact avec la tige 3, et deux extensions 17 en saillie radiale vers l'extérieur, faisant massivement corps avec elle. Les saillies 17 s'étendent en majeure partie à l'extérieur du périmètre de la bague 8 et prolongent vers l'extérieur lesdites surfaces de contact 16.

Les extensions 17 se raccordent à la bague 8 par des parois arrondies 18, à rayon de courbure relativement important.

Les surfaces de contact 16 présentent des aspérités en pointe de diamant complémentaires de celles (non représentées) de la tige 3, c'est-à-dire susceptibles de venir s'interpénétrer lorsqu'elles viennent mutuellement en contact.

La bague 8 comprend également une paroi transversale 20, faisant corps avec elle et s'étendant diamétralement par rapport à elle, en dessus des surfaces de contact 16 par rapport à la vis 2. Comme le montre plus particulièrement la figure 1, cette paroi transversale 20 comprend un trou central 21 et délimite latéralement deux passages 22, dont la forme en arc de cercle est précisément ajustée à celle de la section transversale des branches 5.

Le bouchon 9 comprend un filetage complémentaire du taraudage 7 et présente une cavité supérieure polygonale 25, notamment hexagonale, pour l'engagement d'une clef de serrage. Le fond du bouchon 9 comprend un trou central.

Le rivet 10 (non représenté à la figure 1) comprend une tête venant en appui contre la paroi 20, du côté de la vis 2, et une extrémité opposée à cette tête, susceptible d'être engagée dans le trou central de la paroi 20 et du bouchon 9. Cette extrémité est destinée à être sertie dans la cavité hexagonale 25 pour assurer le montage avec possibilité de rotation du bouchon 9 sur la bague 8. La tête du rivet 10 se trouve en retrait des surfaces de contact 16.

En pratique, après implantation des vis pédiculaires 2 dans les vertèbres, la tige 3 est engagée entre les branches 5 des vis 2.

Les différents ensembles 4 sont alors engagés sur les têtes 2a des vis 2, les branches 5 venant s'engager dans les passages 22.

L'ajustement de ces derniers à la section des branches 5 permet d'immobiliser complètement la bague 8 en rotation sur les branches 5 lors de cet engagement, et d'obtenir un parfait positionnement des surfaces d'appui 16 par rapport à la vis 2, ainsi qu'un parfait positionnement axial du filet du bouchon 9 par rapport au filet des parois taraudées des branches 5.

Les bouchons 9 sont ensuite manoeuvrés dans le sens du vissage, au moyen d'une clef engagée dans leur cavité 25, pour mettre leur filetage en prise avec les taraudages 7.

Au cours de ce vissage, comme le montre la figure 2, les parois 16 viennent porter contre la tige 3 et la presser contre les parois délimitant le fond du canal 6. Dans chaque vis, la bague 8 vient ainsi au contact de la tige 3 non pas sensiblement dans l'axe de vissage du bouchon 9, mais par les deux surfaces de contact latérales 16, de dimensions importantes et qui sont relativement distantes l'une de l'autre.

Grâce à cette conformation spécifique, le dispositif permet d'assurer une parfaite immobilisation de la tige 3 par rapport aux vis 2, susceptible de résister aux contraintes répétées qu'exerce la tige 3 sur les vis 2 lors des différents mouvements du rachis, et ce sans aucun risque de glissement de la tige 3 par rapport aux vis 2.

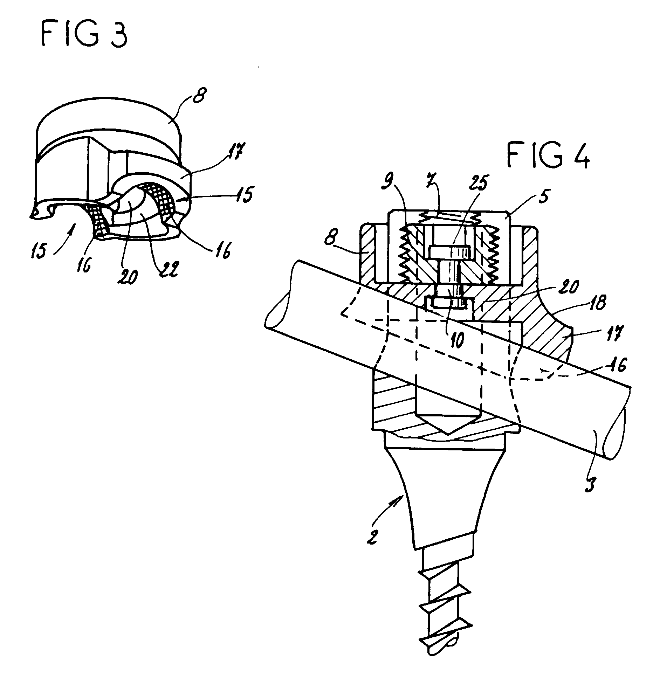

Les figures 3 et 4 montrent une deuxième forme de réalisation de l'invention. Les éléments déjà décrits en référence aux figures 1 et 2 qui se retrouvent dans cette deuxième forme de réalisation sont désignés par les mêmes références numériques.

Comme le montre la figure 4, le canal 6, les surfaces de contact 16 et l'extension 17 de la bague 8 sont conformés de manière à ce que leur axe ne soit pas perpendiculaire à l'axe de vissage du bouchon 9, comme cela était précédemment le cas, mais soit orienté selon un angle différent de 90° par rapport à cet axe de vissage, correspondant à l'orientation de la tige 3 par rapport à la vis 2. Dans ce cas, la bague 8 comprend essentiellement une seule extension 17 et un évidement concave aménagé dans la face inférieure de la paroi transversale 20 pour recevoir la tige 3.

Ainsi, les surfaces de contact 16 que délimitent l'extension 17 et l'évidement concave précité sont conformées de manière à pouvoir venir s'appliquer sur une tige 3 non perpendiculaire à l'axe de vissage du bouchon 9. Plusieurs vis 2 ou bagues 8 comprenant des canaux 6, extensions 17 ou évidements concaves d'inclinaisons différentes par rapport audit axe de vissage sont prévues et peuvent être utilisées alternativement selon les différentes inclinaisons des tiges 3 qui peuvent se rencontrer.

Bien entendu, l'invention n'est pas limitée aux formes de réalisation décrites ci-dessus à titre d'exemples, mais en embrasse au contraire toutes les variantes de réalisation.

Ainsi, la bague 8 peut ne comprendre qu'une seule extension 17, même lorsque la tige 3 est perpendiculaire à l'axe de la vis 2.

## Revendications

1. Dispositif d'ostéosynthèse pour rachis, du type comprenant des éléments d'ancrage osseux (2) et au moins une tige d'étayage (3), chaque élément d'ancrage (2) présentant deux branches latérales parallèles (5) qui délimitent entre elles un canal (6) de réception de la tige d'étayage (3), et recevant une bague (8) engagée autour des branches (5) ainsi qu'un bouchon (9), vissable entre les branches (5),
la bague (8) étant conformée de telle sorte que le bouchon (9) porte sur elle lors de son vissage, afin que le serrage du bouchon (9) permette à la bague (8) de serrer la tige (3) entre elle et le fond du canal (6) recevant la tige (3),
la bague (8) comprenant deux encoches (15) diamétralement opposées, délimitant deux surfaces concaves (16) de contact avec la tige (3); dispositif caractérisé
en ce que la bague (8) comprend au moins une extension (17) en saillie radiale vers l'extérieur, faisant corps avec la bague (8), qui s'étend en majeure partie à l'extérieur du périmètre de la bague (8), au-delà des extrémités latérales des branches (5), et qui prolonge vers l'extérieur l'une au moins desdites surfaces de contact (16).

2. Dispositif selon la revendication 1, caractérisé en ce que le bouchon fileté (9) est assemblé à la bague (8) dans la position qu'il est destiné à occuper par rapport à celle-ci après mise en place sur le rachis.

3. Dispositif selon la revendication 2, caractérisé en ce que la bague (8) comprend une paroi transversale (20), faisant corps avec elle et s'étendant diamétralement par rapport à elle, en dessus de la ou desdites surfaces de contact (16) avec la tige (3) par rapport à l'élément d'ancrage (2), le bouchon (9) étant monté sur cette paroi (20).

4. Dispositif selon la revendication 3, caractérisé en ce que les passages (22) prévus de part et d'autre de la paroi transversale (20) pour l'engagement des branches (5) de l'élément d'ancrage (2) au travers de la bague (8) sont ajustés à la section transversale de ces branches (5).

5. Dispositif selon l'une des revendications 1 à 4, caractérisé en ce que la ou lesdites extensions (17) se raccordent à la bague (8) par des parois arrondies (18).

6. Dispositif selon l'une des revendications 1 à 5, caractérisé en ce que la tige (3) et les surfaces (16) de contact de la bague (8) avec elle présentent des aspérités de surface venant intimement coopérer les unes avec les autres lors du serrage de la bague (8), ou un moletage.

7. Dispositif selon l'une des revendications 1 à 6, caractérisé en ce que les canaux (6) des éléments d'ancrage (2), les surfaces de contact (16) précitées et la ou les extensions (17) de la bague (8) sont conformés de manière à ce que leur axe ne soit pas perpendiculaire à l'axe de vissage du bouchon (9), afin de pouvoir venir s'appliquer sans porte-à-faux sur une tige (3) non perpendiculaire à l'élément d'ancrage, plusieurs éléments d'ancrage (2) ou bagues (8) comprenant des canaux (6), surfaces de contact et extensions (17) d'inclinaisons différentes par rapport audit axe étant prévus et pouvant être utilisés alternativement selon les cas.

## Patentansprüche

1. Vorrichtung zur Wirbelsäulen-Osteosynthese, welche Knochen-Verankerungselemente (2) und mindestens eine Abstützungsstange (3) aufweist, wobei jedes Verankerungselement (2) zwei parallele seitliche Schenkel (5) aufweist, die zwischen ihnen einen Kanal (6) zur Aufnahme der Abstützungsstange (3) begrenzen, und einen Ring (8), der um die Schenkel (5) herum aufgepasst ist, sowie einen Stopfen (9), der zwischen den Schenkeln (5) schraubbar ist, aufnimmt,
wobei der Ring (8) derart ausgebildet ist, dass der Stopfen (9) beim Schrauben auf ihm aufliegt, so dass das Spannen des Stopfens (9) es dem Ring (8) ermöglicht, die Stange zwischen ihm und dem Boden des die Stange (3) aufnehmenden Kanals (6) einzuspannen,
wobei der Ring (8) zwei diametral gegenüberliegende Vertiefungen (15) aufweist, die zwei konkave Flächen (16) zur Berührung mit der Stange (3) begrenzen,
dadurch **gekennzeichnet,** dass
der Ring (8) mindestens eine nach außen hin radial hervorstehende und mit dem Ring (8) einstückige Ausweitung (17) aufweist, die sich größtenteils außerhalb des Umfangs des Rings (8) über die seitlichen Enden der Schenkel (5) erstreckt und die die mindestens eine der Berührungsflächen (16) nach außen hin verlängert.

2. Vorrichtung nach Anspruch 1, dadurch gekennzeichnet, dass der Gewindestopfen (9) mit dem Ring (8) in derjenigen Stellung zusammengefügt wird, die er (9) nach dem Anbringen an der Wirbelsäule in Bezug auf ihn (8) einnehmen soll.

3. Vorrichtung nach Anspruch 2, dadurch gekennzeichnet, dass der Ring (8) eine mit ihm einstückige und sich zu ihm diametral erstreckende Querwand (20) oberhalb der einen oder mehreren Flächen (16) zur Berührung mit der Stange (3) bezüglich des Verankerungselements (2) aufweist, wobei der Stopfen (9) auf dieser Wand (20) gelagert ist.

4. Vorrichtung nach Anspruch 3, dadurch gekennzeichnet, dass die Durchtritte (22), die beiderseits der Querwand (20) zum Einführen der Schenkel (5) des Verankerungselements (2) durch den Ring (8) hindurch vorgesehen sind, an den Querschnitt der Schenkel (5) angepasst sind.

5. Vorrichtung nach einem der Ansprüche 1 bis 4, dadurch gekennzeichnet, dass die eine oder mehreren Ausweitungen (17) sich an den Ring (8) über abgerundete Wände (18) anschließen.

6. Vorrichtung nach einem der Ansprüche 1 bis 5, dadurch gekennzeichnet, dass die Stange (3) und die sie berührenden Flächen (16) des Rings (8) Oberflächen-Rauhigkeiten, die beim Spannen des Rings (8) miteinander innig zusammenwirken, oder eine Rändelung aufweisen.

7. Vorrichtung nach einem der Ansprüche 1 bis 6, dadurch gekennzeichnet, dass die Kanäle (6) der Verankerungselemente (2), die Berührungsflächen (16) und die eine oder mehreren Ausweitungen (17) des Rings (8) derart ausgebildet sind, dass ihre Achse zur Schraubachse des Stopfens (9) nicht senkrecht ist, so dass sie ohne vorzustehen auf einer zum Verankerungselement nicht senkrechten Stange (3) aufliegen können, wobei mehrere Verankerungselemente (2) oder Ringe (8), Kanäle (6), Berührungsflächen (16) und Ausweitungen (17) unterschiedlicher Neigungen bezüglich der Achse vorgesehen sind und je nach Bedarf alternativ verwendet werden können.

## Claims

1. Osteosynthesis device for the spine, of the type comprising elements (2) for anchoring in the bone and at least one supporting rod (3), each anchoring element (2) having two parallel lateral branches (5) which delimit between them a channel (6) to receive the supporting rod (3), and which receive a ring (8) engaged around the branches (5) as well as a cap (9) which can be screwed between the branches (5),
the ring (8) being shaped in such a way that the cap (9) bears on it as it is being screwed on so that the tightening of the cap (9) permits the ring (8) to grip the rod (3) between it and the bottom of the channel (6) which receives the rod (3),
the ring (8) comprising two diametrically opposed recesses (15) which delimit two concave surfaces (16) for contact with the rod (3),
this device being characterised in that the ring (8) comprises at least one extension (17) which projects radially towards the exterior, is integral with the ring (8) and extends for the most part outside the perimeter of the ring (8), beyond the lateral ends of the branches (5), and which extends at least one of the said contact surfaces (16) towards the exterior.

2. Device according to Claim 1, characterised in that the threaded cap (9) is assembled with the ring (8) in the position which it is intended to occupy with respect to the latter after being put in place on the spine.

3. Device according to Claim 2, characterised in that the ring (8) comprises a transverse wall (20) which is integral with it and extends diametrically with respect to it, above the said surface(s) (16) for contact with the rod (3) relative to the anchoring element (2), the cap (9) being mounted on this wall (20).

4. Device according to Claim 3, characterised in that the passages (22) provided on either side of the transverse wall (20) for the engagement of the branches (5) of the anchoring element (2) through the ring (8) are adapted to the cross-section of these branches (5).

5. Device according to one of Claims 1 to 4, characterised in that the said extension(s) (17) is/are joined to the ring (8) by rounded walls (18).

6. Device according to one of Claims 1 to 5, characterised in that the rod (3) and the surfaces (16) of the ring (8) which come into contact therewith have rough areas on the surface which come into close-co-operation with one another as the ring (8) is tightened, or a knurling.

7. Device according to one of Claims 1 to 6, characterised in that the channels (6) of the anchoring elements (2), the aforementioned contact surfaces (16) and the extension(s) (17) of the ring (8) are shaped in such a way that their axis is not perpendicular to the axis of screwing of the cap (9) in order to be capable of being applied without cantilever against a rod (3) which is not perpendicular to the anchoring element, wherein a plurality of anchoring elements (2) or rings (8) comprising channels (6), contact surfaces and extensions (17) having different inclinations with respect to the said axis are provided and can be used alternatively as the case requires.
